# EUROPÄISCHE PATENTSCHRIFT

(11) **EP 1 176 422 B1**
(45) Veröffentlichungstag und Bekanntmachung des Hinweises auf die Patenterteilung: **06.10.2004**
(21) Anmeldenummer: 00116342.7
(22) Anmeldetag: 27.07.2000
(51) Int. Cl.: G01N 33/52

(54) **Sensor-Chips mit Polysiloxan-Mehrfachschichten**
Sensor-chips with polysiloxane multilayers
Puces détectrices à multicouches composées de polysiloxanes

(43) Veröffentlichungstag der Anmeldung: 30.01.2002
(73) Patentinhaber: Micronas Holding GmbH, 79108 Freiburg (DE)
(72) Erfinder: Rühe, Jürgen, 79653 Eichstetten (DE); Klapproth, Holger, 79108 Freiburg (DE); Mohry, Sonja, 79194 Freiburg (DE)
(74) Vertreter: Stürken, Joachim

(56) Entgegenhaltungen:
- WO-A-98/31839
- US-A- 5 661 092
- US-A- 5 667 928
- US-A- 5 696 314

## Beschreibung

Die Erfindung betrifft Sensor-Chips mit Polysiloxan-Mehrfachschichten mit verbesserter Homogenität.

In den letzten Jahren haben in der Analytik Mikrotechniken immer mehr an Bedeutung gewonnen und es sind zahlreiche Festphasensysteme auf der Grundlage von sich selbst organisierenden Monoschichten (engl. "*self-assembled monolayers*", "SAMs") aus bifunktionellen Molekülen (engl. "*Linker*") entwickelt worden, über die spezifisch Probenmoleküle an die Oberfläche des festen Trägers gekoppelt bzw. konjugiert werden, an der dann auch der Nachweis mit Hilfe von geeigneten Markierungen (beispielsweise radioaktiv, gefärbt, fluoreszierend) erfolgt.

Für diese Systeme hat sich in Analogie zu den elektronischen Mikro-Chips die Bezeichnung Sensor-Chips eingebürgert. Im Falle der Konjugation von biologischen Molekülen (sog. "Biokonjugation") an solche Sensor-Chips, beispielsweise Oligonucleotiden oder Antikörpern, spricht man auch von Bio-Chips. Die Kopplung an die Trägeroberfläche kann direkt oder indirekt erfolgen. Ein Beispiel für eine indirekte Kopplung ist die Kopplung einer nachzuweisenden Nucleinsäuresequenz durch Hybridisierung an ein immobilisiertes, komplementäres Oligonucleotid als Sonde. In diesem Fall hat die Verwendung der Sonde noch den Vorteil der natürlichen Spezifität der Wechselwirkung biologischer Makromoleküle.

Typischerweise werden zur Herstellung von Sensor-Chips Oberflächen aus Metall- bzw. Halbmetalloxiden, wie z.B. Aluminiumoxid, Quarzglas, Glas, in eine Lösung von bifunktionellen Molekülen (sog. "Linker"), die beispielsweise eine Halogensilan- (z.B. Chlorsilan-) oder Alkoxysilangruppe zur Kopplung an die Trägeroberfläche aufweisen, getaucht, so daß sich eine sich selbst organisierende Monoschicht (SAM) bildet. Diese weist in diesem Fall eine Dicke von wenigen Ångström aus. Die Kopplung der Linker an die Proben- oder Sondenmoleküle erfolgt über eine geeignete weitere funktionelle Gruppe, beispielsweise eine Amino- oder Epoxygruppe. Geeignete bifunktionelle Linker für die Kopplung einer Vielzahl von Probenoder Sonden-Molekülen, insbesondere auch biologischen Ursprungs, an eine Vielzahl von Trägeroberflächen sind dem Fachmann gut bekannt, vgl. beispielsweise "Bioconjugate Techniques" von G. T. Hermanson, Academic Press 1996.

Eine homogene und chemisch resistente Beschichtung läßt sich mit diesem Verfahren aber nur sehr schwer erzielen. Monofunktionelle Silane, die sich für feste Träger mit Metalloxidoder Halbmetalloxidoberflächen eignen, z.B. Monochlorsilane wie Aminopropylmonochlorsilan, ergeben zwar echte Monolagen, lassen sich aber bereits durch heißes Wasser von der Oberfläche abspalten. Trifunktionelle Silane, wie z.B. Aminopropyltriethoxysilan, bilden bereits in der Lösung Netzwerke aus, die dann zu einer inhomogenen Beschichtung der Trägeroberfläche aus Silanen führen. Eine homogene Beschichtung ergibt sich somit nicht, was Qualitätseinbußen zur Folge hat. Hinzu kommen auch noch Chargenunterschiede innerhalb einer Chip-Serie.

Ein weiterer Nachteil dieses Verfahrens bei der praktischen Anwendung ist die relativ lange Inkubationzeit in der Silanlösung. Für eine effiziente Beschichtung sind oftmals Silanisierungszeiten von ca. zwei Stunden erforderlich.

Aufgabe der Erfindung ist, ausgehend von diesem Stand der Technik, die Bereitstellung eines Sensor-Chips mit einer homogenen Beschichtung auf Basis eines Silan-Linkers sowie die Angabe eines Verfahrens, mit dem diese rasch und reproduzierbar herstellbar ist.

Die Erfindung beruht auf der Erkenntnis, daß die herkömmlichen Silan-Monoschichten durch Polysiloxan-Mehrfachschichten ersetzbar sind, die sich mit an sich bekannten Beschichtungsverfahren sehr homogen und vor allen Dingen rasch herstellen lassen.

Die Erfindung betrifft somit gemäß Patentanspruch 1 einen Sensor-Chip.

Die erfindungsgemäßen Sensor-Chips zeichnen sich durch eine bessere Homogenität der Polysiloxan-Mehrfachschicht und somit eine bessere Reproduzierbarkeit und Verarbeitbarkeit aus. Dadurch verbessert sich auch deutlich die Reproduzierbarkeit der Sensoreigenschaften. Ferner ist der Durchsatz höher, und das "Altern" der Lösung des bifunktionellen Silans stellt kein Problem mehr dar.

Vorteilhafte und/oder bevorzugte Ausführungsformen der Erfindung sind Gegenstand der Unteransprüche.

Die Erfindung betrifft einen Sensor-Chip, auf dessen Metalloxid- oder Halbmetalloxidoberfläche eine homogene Polysiloxan-Mehrfachschicht aufgebracht ist, die erhältlich ist, indem der Sensor-Chip in eine Lösung eines bifunktionellen Silans in einem Lösungsmittel mit einer Siedetemperatur im Bereich von 50 bis 150°C, vorzugsweise 60 bis 90°, beispielsweise Ethanol, Chloroform oder Toluol, mit einer Konzentration im Bereich von 0,1 bis 50 Gew.-%, beispielsweise 0,5 bis 40 Gew.-%, vorzugsweise 1,0 bis 30 Gew.-% oder 2 bis 25 Gew.-% oder 3 bis 20 Gew.-% oder 5 bis 15 Gew.-%, vorzugsweise Raumtemperatur eingetaucht und dann mit einer Geschwindigkeit im Bereich von 0,1 bis 10 mm/s, beispielsweise 0,5 bis 8 mm oder 1 bis 5 mm oder 2 bis 3 mm, wieder herausgezogen wird, worauf die Schicht aus dem bifunktionellen Silan unter Bildung der Polysiloxan-Mehrfachschichten auf der Oberfläche des Sensor-Chips durch Vernetzenkovalent fixiert wird.

Bei dieser Ausführungsform spielen weder die Eintauchtiefe noch die Eintauchgeschwindigkeit oder die Verweildauer eine Rolle.

Im folgenden wird die Erfindung ohne Beschränkung unter Bezugnahme auf konkrete Ausführungsformen und Beispiele detaillierter erläutert.

Die Oberfläche des erfindungsgemäßen Sensor-Chips kann aus einem beliebigen Metalloxid oder Halbmetalloxid oder einem anderen Träger bestehen, das/der mit Silangruppen unter Bildung von kovalenten Bindungen reagiert. Beispielsweise kann es sich um Al₂O₃- oder SiO₂-haltige Oberflächen handeln, z.B. um Glas oder Quarzglas ("fused silica"). Eine SiO₂-haltige Oberfläche kann auch hergestellt werden, indem eine Schicht aus hochdispersem Siliciumdioxid ("fumed silica") auf einen beliebigen festen Träger aufgebracht oder Schichten aus SiOₓ auf einen festen Träger aufgedampft oder gesputtert werden.

Das verwendete bifunktionelle Silan unterliegt keinen besonderen Beschränkungen, sofern es unter Bildung von Polysiloxanen vernetzt werden kann. In Frage kommen alle Silane, die am Siliciumatom ein(e), zwei oder drei hydrolysierbare(s) Atom(e) oder Gruppe(n) aufweisen, beispielsweise Halogenatome, C₁-C₄-Alkoxy-, C₁-C₄-Acyloxy- oder Aminogruppen.

Die zweite funktionelle Gruppe des bifunktionellen Silans unterliegt ebenfalls keinen besonderen Beschränkungen und wird nach Maßgabe des zu immobilisierenden Proben- oder Sondenmoleküls gewählt. Beispiele sind zu nucleophilen Substitutionsreaktionen, nucleophilen Additionsreaktionen, Diels-Alder-Reaktionen oder radikalischen Substitutionsreaktionen fähige reaktive Gruppen. Konkrete Beispiele sind reaktive Doppelbindungen, Diengruppen, dienophile Gruppen, Epoxy-, Aldehyd- Hydroxy-, Carbonsäure-, Aktivester-, Amino, Disulfid-, Thiol-, Aziridin-, Azlacton-, Isocyanat-, Isothiocyanat-, Azidgruppen.

Konkret kann zum Beispiel Isocyanopropyltrimethoxysilan oder Glycidoxypropyltrimethoxysilan auf einen Glasträger aufgebracht werden.

Zur homogenen Beschichtung der Oberfläche des Sensor-Chips mit dem bifunktionellen Silan kommt beispielsweise die Tauchbeschichtung in Frage, die seit langem in der Elektronikindustrie angewendet wird, um z.B. Chip-Wafer oder Festplattenmedien schnell mit hoher Präzision zu beschichten. Es kommen aber auch andere Verfahren in Frage, die eine homogene Beschichtung gewährleisten. Beispielsweise kann die Beschichtung auch durch Aufschleudern, Rakeln (Aufbringen mit dem Streichmesser), Aufsprühen oder Aufstreichen (Lackieren) erfolgen. Die Tauchbeschichtung wird erfindungsgemäß besonders bevorzugt, weil die Schichtdicke (im Prinzip beliebig, z.B. einige 100 Å) einfach über die Konzentration des bifunktionellen Silans das verwendete Lösungsmittel, die Temperatur und die Tauchgeschwindigkeit eingestellt werden kann. Durch Beimischung von Präpolymeren als Viskositätserhöher kann die Viskosität der Lösung auf einen geeigneten Wert eingestellt werden, z.B. kann ein Polydimethylsiloxan mit geeigneten kondensierbaren Gruppen, beispielsweise Silanolgruppen, Chlorsylylgruppen oder Alkoxysilylgruppen, verwendet werden.

Das Lösungsmittel für die bifunktionellen Silane unterliegt keinen besonderen Beschränkungen, sofern es schnell genug verdampfbar ist. Beispielsweise eignet sich Chloroform, Ethanol oder Toluol.

Die Tauchbeschichtung eines Trägers kann in 5 Minuten erfolgen. Zum Vergleich dauert die Beschichtung unter Bildung von SAMs nach den Verfahren des Standes der Technik ca. 2h.

Ein weiterer Vorteil ist, daß die Tauchbeschichtung in hohem Grade im Parallelbetrieb erfolgen kann. Beispielsweise kann ein einfacher Halter zum Eintauchen verwendet werden, bei dem sich z.B. 30 handelsübliche Objektträger (feste Phase des späteren Sensor-Chips) in beispielsweise zwei Reihen mit der schmalen Oberseite bzw. Griff-Fläche in entsprechend vielen Halteschlitzen befinden. Diese Halteschlitze sind der Breite (der schmalen Oberseite) und Dicke der Objektträger angepaßt und können außerdem auf einer oder beiden schmalen Seiten eine Einrichtung zum Fixieren des Objektträgers in dem Halteschlitz aufweisen, beispielsweise eine gefederte Metallkugel, mit der der Objektträger angepresst wird. Die Eintauchtiefe beträgt zum Beispiel 60 mm.

Mit diesem Halter lassen sich z.B. 30 Träger gleichzeitig tauchbeschichten. Die sonst üblichen Waschschritte sind unnötig und auch das Trockenblasen der Träger entfällt.

Da keine Kontaktflächen (außer der Griff-Fläche der Objektträger) vorhanden sind, kann die Flüssigkeit beim Herausziehen ohne die Bildung von Tropfen/"Nasen", welche die Homogenität der Beschichtung stören könnten, ablaufen. Dadurch wird der Silanisierungsprozess nicht nur verbessert, sondern auch beschleunigt.

Nach der Aufbringung wird die Schicht aus dem bifunktionellen Silan unter Bildung der Polysiloxan-Mehrfachschichten auf der Oberfläche des Sensor-Chips fixiert (durch Vernetzung), beispielsweise indem sie Luftfeuchtigkeit ausgesetzt wird. Gegebenenfalls kann zusätzlich im Trockenschrank bei erhöhter Luftfeuchtigkeit erwärmt werden, z.B. bei 120°C.

Die folgende Tabelle zeigt einen Vergleich zwischen einem herkömmlicherweise und dem erfindungsgemäß angewendeten Verfahren (Silanisierung von 150 Objektträgern).

| | Aktivieren | Silanisieren | Spülen/ Trockenblasen | Fixieren der Silanschicht |
|---|---|---|---|---|
| Herkömmlich | 2h | 2h | 2h | 2h bei 120°C |
| Erfindung | entfällt | 5 min | entfällt | 2h bei 120°C |

Es folgt ein Ausführungsbeispiel:

In den oben beschriebenen Halter wurden 30 Glasobjekträger (als spätere Sensor-Chips) mit der Griff-Fläche (schmale Oberseite) nach oben sauber eingespannt. Die Objektträger wurden in 500 ml heiße Hellmanex®-Lösung (Detergenzlösung zum Reinigen von Fa. Hellma) eingetaucht (10 min) und anschließend 3 x 2 Minuten in mit einer Millipore®-Anlage entionisiertem Wasser gewässert und dann 3 Minuten in Ethanol gebadet. Anschließend wurden die Objektträger getrocknet (Trokkenschrank) oder direkt in 1 Gew.-% Glycidoxypropyltrimethoxysilan-Lösung (Toluol als Lösungsmittel) getaucht. Die Eintauchgeschwindigkeit betrug 2,5 mm/s und die Herausziehgeschwindigkeit 0,5 mm/s. Nach den Herausziehen können die Objektträger direkt im Trockenschrank bei 120 °C fixiert werden.

## Patentansprüche

1. Sensor-Chip mit einer Trägeroberfläche aus Metalloxid oder Halbmetalloxid, auf die eine homogene Polysiloxan-Mehrfachschicht aufgebracht ist, die erhältlich ist, indem der Sensor-Chip in eine Lösung eines bifunktionellen Silans in einem Lösungsmittel mit einer Siedetemperatur im Bereich von 50 bis 150°C mit einer Konzentration im Bereich von 0,1 bis 50 Gew.-% eingetaucht und dann mit einer Geschwindigkeit im Bereich von 0,1 bis 10 mm/s wieder herausgezogen wird, worauf die Schicht aus dem bifunktionellen Silan unter Bildung der Polysiloxan-Mehrfachschichten auf der Oberfläche des Sensor-Chips durch Vernetzenkovalent fixiert wird.

2. Sensor-Chip nach Anspruch 1, wobei es sich bei der Trägeroberfläche aus Metalloxid oder Halbmetalloxid um eine Al₂O₃- oder SiO₂-haltige Oberfläche handelt.

3. Sensor-Chip nach Anspruch 2, wobei die SiO₂-haltige Trägeroberfläche Glas oder Quarzglas oder eine Schicht aus hochdispersem Siliciumdioxid auf einen beliebigen festen Träger aufgebracht oder Schichten aus SiOₓ auf einen festen Träger aufgedampft oder gesputtert umfaßt.

4. Sensor-Chip nach einem der vorstehenden Ansprüche, wobei das bifunktionelle Silan am Siliciumatom ein(e), zwei oder drei hydrolysierbare(s) Atom(e) oder Gruppe(n) aufweist.

5. Sensor-Chip nach Anspruch 4, wobei die hydrolysierbaren Atome oder Gruppen Halogenatome, C₁-C₄-Alkoxy-, C₁-C₄-Acyloxy- oder Aminogruppen umfassen.

6. Sensor-Chip nach einem der vorstehenden Ansprüche, wobei die zweite funktionelle Gruppe des bifunktionellen Silans nucleophile Substitutionsreaktionen, nucleophile Additionsreaktionen, Diels-Alder-Reaktionen oder radikalische Substitutionsreaktionen eingehen kann.

7. Sensor-Chip nach Anspruch 6, wobei es sich bei der zweiten funktionellen Gruppe des bifunktionellen Silans um eine reaktive Doppelbindung, Diengruppe, dienophile Gruppe, Epoxy-, Aldehyd-, Hydroxy-, Carbonsäure-, Aktivester-, Amino-, Disulfid-, Thiol-, Aziridin-, Azlacton-, Isocyanat-, Isothiocyanat- oder Azidgruppe handelt.

## Claims

1. Sensor chip comprising a substrate surface of metal oxide or semimetal oxide to which a homogeneous polysiloxane multilayer has been applied, which is obtainable by immersing the sensor chip in a solution of a bifunctional silane in a solvent having a boiling point in the range from 50 to 150°C with a concentration in the range from 0.1 to 50% by weight and then drawing said chip out again at a speed in the range from 0.1 to 10 mm/s, after which the layer of the bifunctional silane is covalently fixed on the surface of the sensor chip by crosslinking with formation of the polysiloxane multilayers.

2. Sensor chip according to Claim 1, the substrate surface of metal oxide or semimetal oxide being an Al₂O₃- or SiO₂-containing surface.

3. Sensor chip according to Claim 2, the SiO₂- containing substrate surface comprising glass or quartz glass or a layer of highly disperse silica applied to any desired solid substrate or layers of SiOₓ applied to a solid substrate by vapour deposition or sputtering.

4. Sensor chip according to any of the preceding Claims, the bifunctional silane having one hydrolysable atom or a group or two or three hydrolysable atoms or groups or the silicon atom.

5. Sensor chip according to Claim 4, the hydrolysable atoms or groups comprising halogen atoms or C₁-C₄-alkoxy, C₁-C₄-acyloxy or amino groups.

6. Sensor chip according to any of the preceding Claims, it being possible for the second functional group of the bifunctional silane to undergo nucleophilic substitution reactions, nucleophilic addition reactions, Diels-Alder reactions or free radical substitution reactions.

7. Sensor chip according to Claim 6, the second functional group of the bifunctional silane being a reactive double bond, diene group, dienophilic group or epoxy, aldehyde, hydroxyl, carboxyl, active ester, amino, disulphide, thiol, aziridine, azlactone, isocyanate, isothiocyanate or azide group.

## Revendications

1. Puce de détection présentant une surface de substrat en oxyde métallique ou en oxyde de semi-métal sur laquelle une couche multiple homogène en polysiloxane est appliquée, laquelle peut être obtenue en immergeant la puce de détection dans une solution d'un silane bifonctionnel dans un solvant présentant une température d'ébullition s'inscrivant dans une plage de 50 à 150 °C avec une concentration s'inscrivant dans une plage de 0,1 à 50 % en poids, puis en la retirant de nouveau à une vitesse s'inscrivant dans une plage de 0,1 à 10 mm/s, la couche en silane bifonctionnel étant fixée de façon covalente par réticulation en formant les couches multiples en polysiloxane sur la surface de la puce de détection.

2. Puce de détection selon la revendication 1, dans laquelle la surface du substrat en oxyde de métal ou en oxyde de semi-métal est une surface contenant du Al₂O₃ ou SiO₂.

3. Puce de détection selon la revendication 2, dans laquelle la surface du substrat contenant du SiO₂ comprend du verre ou du verre de quartz ou une couche en dioxyde de silicium fortement dispersée appliqué sur un substrat solide quelconque ou des couches de SiOₓ appliquées par dépôt de vapeur chimique ou pulvérisées sur un substrat solide.

4. Puce de détection selon l'une quelconque des revendications précédentes, dans laquelle le silane bifonctionnel présente au niveau de l'atome de silicium un, deux ou trois atomes ou groupes hydrolysables.

5. Puce de détection selon la revendication 4, dans laquelle les atomes ou groupes hydrolysables comprennent des atomes d'halogène, les groupes alcoxy en C₁-C₄, acyloxy en C₁-C₄ ou amino.

6. Puce de détection selon l'une quelconque des revendications précédentes, dans laquelle le second groupe fonctionnel du silane bifonctionnel peut participer à des réactions de substitution nucléophile, des réactions d'addition nucléophile, des réactions de Diels-Alder ou des réactions de substitution radicalaire.

7. Puce de détection selon la revendication 6, dans laquelle le second groupe fonctionnel du silane bifonctionnel est une double liaison réactive, un groupe diène, un groupe diénophile, des groupes époxy, aldéhyde, hydroxy, acide carboxylique, ester actif, amino, disulfure, thiol, aziridine, azlactone, isocyanate, isothiocyanate ou azide.
